# EUROPEAN PATENT SPECIFICATION

(11) **EP 4 082 485 B1**
(45) Date of publication and mention of the grant of the patent: **31.01.2024**
(21) Application number: 21170757.5
(22) Date of filing: 27.04.2021
(51) Int. Cl.: A61F 2/38, A61F 2/46, A61F 2/30, A61B 17/02

(54) **TIBIAL TRIAL INSERT SYSTEM**
TIBIALES PROBEEINSATZ-SYSTEM
SYSTÈME D'INSERTION D'ESSAI TIBIAL

(43) Date of publication of application: 02.11.2022
(73) Proprietor: Aesculap AG, 78532 Tuttlingen (DE)
(72) Inventor: Zouaghi, Housseyn, 57070 Metz (FR)
(74) Representative: Patentanwälte Ruff, Wilhelm, Beier, Dauster & Partner mbB

(56) References cited:
- WO-A1-2013/024245
- US-A- 5 733 292
- US-A1- 2012 158 152
- US-A1- 2013 138 112
- US-A1- 2013 261 759
- US-A1- 2014 277 543
- US-A1- 2016 346 098

## Description

### TECHNICAL FIELD AND PRIOR ART

The invention relates to a tibial trial insert system and is defined in claim 1. During a total knee arthroplasty, tibial trial insert systems are typically used to assist a surgeon in determining a size, shape or other configuration of a permanent prosthesis that is designed to replace a portion of the knee joint. In particular, such tibial trial insert systems are used to determine a relative spacing between a femoral component and a tibial component of the permanent prosthesis.

US 2015/0359642 A1 discloses a tibial trial insert system comprising a bearing component having a superior articulating surface and an inferior surface; a base component having a superior surface and an inferior surface, the base component configured to removably engage with the bearing component; and a plurality of shims, each shim configured to be slidable between the inferior surface of the bearing component and the superior surface of the base component to change a relative proximal/distal spacing between the bearing component and the base component.

US 2014/277543 A1 discloses a tibial trial insert system comprising a bearing component having a superior articulating surface and an inferior surface; a plate component having a superior surface and an inferior fixation surface; a spacing adjustment coil arranged between the inferior surface of the bearing component and the superior surface of the plate component, the spacing adjustment coil having at least one superior connector element configured to be welded to the bearing component, at least one inferior base element configured to be welded to the plate component; a plurality of shims, each shim configured to be slidable between an inferior surface of the connector element and a superior surface of the base element to adjust a proximal/distal height of the spacing adjustment assembly.

### SUMMARY OF THE INVENTION

It is an object of the present invention to provide an alternative tibial trial insert system.

According to one aspect, a tibial trial insert system is provided, said tibial trial insert system comprising: a bearing component having a superior articulating surface and an inferior surface; a plate component having a superior surface and an inferior fixation surface; a spacing adjustment assembly configured to be arrangeable between the inferior surface of the bearing component and the superior surface of the plate component, the spacing adjustment assembly having at least one superior connector element configured to removably engage with the bearing component, at least one inferior base element configured to removably engage with the plate component, and having a coupling arrangement movably coupling the connector element with the base element in proximal/distal direction and to a limited extent; a plurality of shims, each shim configured to be slidable between an inferior surface of the connector element and a superior surface of the base element to adjust a proximal/distal height of the spacing adjustment assembly, in order to thereby adjust a relative proximal/distal spacing between the bearing component and the plate component. The solution according to the invention avoids, in particular, incorrect adjustment of the proximal/distal spacing between the bearing component and the plate component. To this end, the spacing adjustment assembly only allows a limited proximal/distal relative movement between the bearing component and the plate component. In order to achieve said limited movement, the connector element and the base element are movably and captively coupled by means of the coupling arrangement. This prevents an insertion of an excessive number of shims and thus the setting of too large a proximal/distal height. As a result, the invention helps to prevent errors during surgery and improves patient safety. Moreover, due to the movable yet captive coupling between the connector element and the base element the spacing adjustment assembly can be handled easily during surgery, in particular in comparison to a mere arrangement of loose and/or non-pre-assembled parts. Preferably, the connector element and the base element are fixed relative to each other by means of the coupling arrangement in anterior/posterior and/or medial/lateral direction. Engagement of the connector element with the bearing component and engagement of the base element with the plate component restrains an anterior/posterior movement and/or a medial/lateral movement between the bearing component and the plate component. For engagement with the base element, the plate component preferably comprises a recess, into which recess a portion of the base element provided for that purpose can be accommodated in anterior/posterior direction and/or medial/lateral direction in a form-fitting manner. For engagement with the connector element, the bearing component preferably comprises an engagement portion which interacts with a complementary engagement portion of the connector element, thereby forming a plug-in, latching and/or snap-in connection. Preferably, the movability between the connector element and the base element in proximal/distal direction is limited by means of an end-stop or the like. In this document the terms "superior", "inferior", "anterior", "posterior", "medial", "lateral", "proximal" and "distal" are used according to their standard anatomical definitions. In this document the phrase "proximal/distal spacing" denotes a spacing that extends in a proximal and/or distal direction. Analogously, the phrase "anterior/posterior" means anterior and/or posterior; the phrase "medial/lateral" means medial and/or lateral. The plate component can also be termed "tibial plateau component".

In one embodiment the coupling arrangement forms a telescopic mechanism which is extendable in proximal direction and retractable in distal direction. In this way, a particularly robust and easy to manufacture configuration of the coupling arrangement is achieved. The telescopic mechanism is telescoping in proximal/distal direction and, in this respect, extendable in the proximal direction and retractable in the distal direction. The telescopic mechanism is fixed to the base element on one end and fixed to the connector element on the other end. The telescopic mechanism is extendable in proximal direction by means of sliding at least one shim of the plurality of shims between the connector element and the base element. Starting from an extended position, the telescopic mechanism is retractable in distal direction by means of removing at least one shim of the plurality of shims between the connector element and the base element.

In one embodiment the telescopic mechanism comprises at least a first cylinder element and a second cylinder element, wherein the first cylinder element is slidably received in a bore of the base element, wherein the second cylinder element is slidably received in a bore of the first cylinder element, and wherein the second cylinder element is at least indirectly connected to the inferior surface of the connector element. The first and second cylinder element form a telescopic cylinder with at least two stages. In further embodiments the telescopic mechanism comprises three, four, five, six or even more cylinder elements, forming a telescopic cylinder with an according number of stages. This embodiment offers a further simplified construction and at the same time a particularly robust coupling between the connector element and the base element and, thus, also between the bearing component and the plate component. Preferably, the bore of the base element and the bore of the first cylinder element are arranged coaxially. The bore of the base element and the bore of the first cylinder element extend in proximal/distal direction. Preferably, the bore of the base element and/or the bore of the first cylinder element is formed as a through-hole. Preferably, the proximal/distal movability of the first cylinder element within the bore of the base element is limited by means of a proximal end-stop and/or a distal end-stop. Preferably, the proximal/distal movability of the second cylinder element within the bore of the first cylinder element is limited by means of a proximal end-stop and/or a distal end-stop.

In one embodiment the first cylinder element and the second cylinder element each have a rotationally asymmetrical, preferably oval, cross-section, in order to thereby prevent a rotation about a proximal/distal axis. The rotationally asymmetrical, preferably oval, cross-section restrains a relative rotation between the connector element and the base element and, thus, between the bearing component and the plate component. This leads to an improved relative positioning between the bearing component and the plate component and avoids errors in determining the size, shape or other configuration of the permanent prosthesis. Provided the telescopic mechanism comprises further cylinder elements, preferably, said further cylinder elements each have a rotationally asymmetrical, preferably oval, cross-section. Preferably, the bore of the base element and/or the bore of the first cylinder element have a rotationally asymmetrical, preferably oval, cross-section, said cross-section being complementary to the asymmetrical cross-section of the first cylinder element and/or the second cylinder element.

In one embodiment the telescopic mechanism is extendable between 0 mm and 16 mm, inclusive. The inventors have found that an extension between 0 mm and 16 mm, inclusive, offers particular advantages.

In one embodiment the tibial trial insert system further comprises a set of different sized plate components and a set of different sized adapter elements, each of the adapter elements being associated with one of the plate components and configured to removably engage with the one of the plate components and with the base element of the spacing adjustment assembly. The set of different sized plate components allows the tibial trial insert system to be adapted to different anatomical characteristics of a patient. In other words, a comparatively large plate component of the set of different sized plate components is used for a comparatively large tibia. A rather small plate component of the set of different sized plate components is used for a rather small tibia. The set of different sized adapter elements allows providing and/or using the same spacing adjustment assembly in connection with the different sized plate components. Preferably, the plate components have different sized recesses to accommodate one of the different sized adapter elements. The different sized adapter elements preferably each comprise a recess, the recesses having identical dimensions for accommodating the base element of the spacing adjustment assembly.

In one embodiment the tibial trial insert system further comprises a set of different sized plate components and a set of different spacing adjustment assemblies having different sized base elements, each of the base elements being associated with one of the plate components and configured to removably engage with the one of the plate components. The different sized plate components allow the tibial trial insert system to be adapted to different anatomical characteristics of the patient. Each of the different spacing adjustment assemblies is associated with one of the plate components. Preferably, the different sized plate components comprise different sized recesses, into which the base elements can be accommodated in a form-fitting manner. With the exception of the size of the base elements, preferably all spacing adjustment assemblies are identical.

In one embodiment the plurality of shims can be stacked one above another and comprises different shims having different thicknesses and different matching portions, each of the matching portions configured for matching with a different complementary matching portion of the plurality of shims and/or the spacing adjustment assembly, in order to thereby prevent a stacking of the plurality of shims in a non-defined stacking order. This can prevent an incorrect adjustment of the proximal/distal height of the spacing adjustment assembly and, thus, the proximal/distal spacing between the bearing component and the plate component. In order to adjust the proximal/distal spacing, at least one shim is used. Moreover, two or more of the plurality of shims can be used in combination to provide for additional spacing between the bearing component and the base component. With other words, the plurality of shims can be stacked one above another. The permanent prostheses are often only available in fixed, different sizes in relation to the proximal/distal distance between the femoral and tibial components of the prostheses. It is therefore desirable to adjust the proximal/distal spacing between the bearing component and the plate component in predefined adjustment steps. Said predefined adjustment steps are achieved by means of specific, well-defined stacking orders of the plurality of shims. This embodiment avoids a stacking of the plurality of shims in a non-defined order and, thus, an adjustment of the proximal/distal spacing that does not correspond with the fixed, different sizes of the permanent prosthesis. In case the shims are stacked one above another in a well-defined stacking order, the different matching portions match, preferably form-fittingly, with the complementary matching portions. In case an operating surgeon should try to slide a shim into a non-defined stacking order, the matching portion of said shim acts as stop, the stop preventing a further sliding of the shim between the base element and the connector element. The actual size and/or shape of the different matching portions prevents the use of a shim that does not correspond to the thicknesses available on the permanent prosthesis. In other words, the size and/or shape of the different matching portions conditions possible stacking orders and, thus, possible incremental adjustment steps.

In one embodiment the different shims of the plurality of shims each comprise a spring-loaded latching portion, each of the latching portions being configured for releasable form- and/or force-fitting connection with a complementary latching portion of the spacing adjustment assembly. The spring-loaded latching portions and the complementary latching portion form a releasable latching connection that helps to avoid an unintended removal of the according shim. Moreover, said latching connection may stabilize the stack of shims and thereby the whole tibial trial insert system. Preferably, the latching portions each comprise at least one spring element. Preferably, the spring elements each comprise a latching protrusion acting as actual latch for form-fitting and/or force-fitting interaction with the complementary latching portion of the spacing adjustment assembly. Preferably, each spring element is in form of, e.g., a leaf spring, beam spring and/or bar spring, wherein the latching protrusion is disposed at an end portion of the spring element. Preferably, the complementary latching portion is disposed on the base element and has the form of a recess for receiving the latching protrusion.

In one embodiment the different thicknesses range between 1 mm and 8 mm, inclusive. The inventors have found that a thickness range between 1 mm und 8 mm offers particular advantages.

In one embodiment the plurality of shims comprises at least one first shim having a first thickness, preferably of 1 mm, at least one second shim having a second thickness, preferably of 2 mm, and at least one third shim having a third thickness, preferably of 4 mm, at least one fourth shim having a fourth thickness, preferably 6 mm, and at least one fifth shim having a fifth thickness, preferably 8mm. This allows for a particularly advantageous adaption of the proximal/distal spacing in predetermined incremental steps. Preferably, the plurality of shims comprises exactly two first shims, three second shims, two third shims, two fourth sims and two fifth shims. This allows for an incremental adjustment of the proximal/distal spacing within a relatively large adjustment range, while at the same time the total number of shims is rather small.

In one embodiment the tibial trial insert system further comprises a shim handling instrument having an instrument shaft and a coupling mechanism distally disposed on the instrument shaft, the coupling mechanism being configured to releasably couple to an anterior coupling portion of each of the plurality of shims. The shim handling instrument allows for a simplified and ergonomic handling of the shims. The instrument shaft extends between a distal end and a proximal end. The coupling mechanism is disposed on the distal end of the instrument shaft. The coupling mechanism can be operated manually. For this purpose, an operating element is preferably provided by means of which the coupling mechanism can be manually shifted between a coupling state and a release state. The operating element is preferably a button, a switch, a slider or the like.

In one embodiment the coupling mechanism comprises a coupling finger extending in proximal/distal direction and being spring-loaded in medial/lateral direction, and wherein the coupling portions each comprise an L-shaped coupling slot for positively receiving the coupling finger, the L-shaped coupling slot extending in anterior/posterior and medial/lateral direction. The L-shaped coupling slot comprises a first portion extending in anterior/posterior direction and a second portion extending in medial/lateral direction. In order to couple with the coupling portion, the coupling finger can be inserted into the first portion and from there into the second portion of the coupling slot. Within the second portion the coupling finger form-fittingly engages with the coupling portion in anterior/posterior direction. This prevents an unintended decoupling of the shim while sliding the shim along the anterior/posterior direction. In the coupling state the spring-load prevents an unintentional decoupling of the coupling finger from the second portion of the L-shaped coupling slot.

### BRIEF DESCRIPTION OF THE DRAWINGS

In the following, an embodiment of the invention will be described in detail with reference to the drawings. Throughout the drawings, the same elements will be denoted by the same reference numerals. The drawings schematically show:
- fig. 1: in a perspective view an embodiment of a tibial trial insert system having a bearing component, a plate component, a spacing adjustment assembly and a plurality of shims, wherein fig. 1 only shows only one exemplary shim of the plurality of shims;
- fig. 2: a perspective exploded view of the spacing adjustment assembly of the tibial trial insert system according to fig. 1;
- fig. 3: a further perspective and partially exploded view of the spacing adjustment assembly;
- fig. 4: a perspective view of the plate component together with the spacing adjustment assembly;
- fig. 5: a perspective view of the plate component together with a variant of the spacing adjustment assembly and an adapter element;
- fig. 6: in a perspective exploded view the plate component, the adapter element and a base element of the spacing adjustment assembly according to fig. 5;
- fig. 7: a perspective view of a configuration of the tibial trial insert system according to fig. 1, wherein first, second, third and fourth shims are stacked one above another between the base element and a connector element of the spacing adjustment assembly;
- fig. 8: a perspective detail view of a first shim with a line of sight directed towards a superior surface of the first shim;
- fig. 9: a further perspective detail view of the first shim with a line of sight directed towards an inferior surface of the shim;
- figs. 10, 11: different perspective detail views of a further shim similar to the views according to figs. 8 and 9, respectively;
- figs. 12, 13: different perspective detail views of a second shim similar to the views according to figs. 8 and 9, respectively;
- figs. 14, 15: different perspective detail views of a third shim similar to the views according to figs. 8 and 9, respectively;
- figs. 15a, 15b: different perspective detail views of a fourth shim similar to the views according to figs. 8 and 9, respectively;
- figs. 15c, 15d: different perspective detail views of a fifth shim similar to the views according to figs. 8 and 9, respectively;
- fig. 15e: a perspective detail view of one of the shims being releasably latched with the spacing adjustment assembly;
- fig. 16: a perspective detail view of a shim handling instrument having an instrument shaft and a coupling mechanism;
- fig. 17: a perspective detail view of the instrument shaft;
- fig. 18: a perspective detail view of the coupling mechanism;
- fig. 19: a perspective view of a further configuration of the tibial trial insert system, wherein the shim handling instrument is used to handle a shim;
- fig. 20: a perspective view of a further configuration of the tibial trial insert system;
- fig. 21: a perspective view of a further configuration of the tibial trial insert system;
- fig. 22: a perspective view of a further configuration of the tibial trial insert system;
- fig. 23: a perspective view of a further configuration of the tibial trial insert system;
- fig. 24: a perspective view of a further configuration of the tibial trial insert system;
- fig. 25: a perspective view of a further configuration of the tibial trial insert system, and
- fig. 26: a perspective view of a set of different sized plate components. DETAILED DESCRIPTION OF EMBODIMENTS OF THE INVENTION

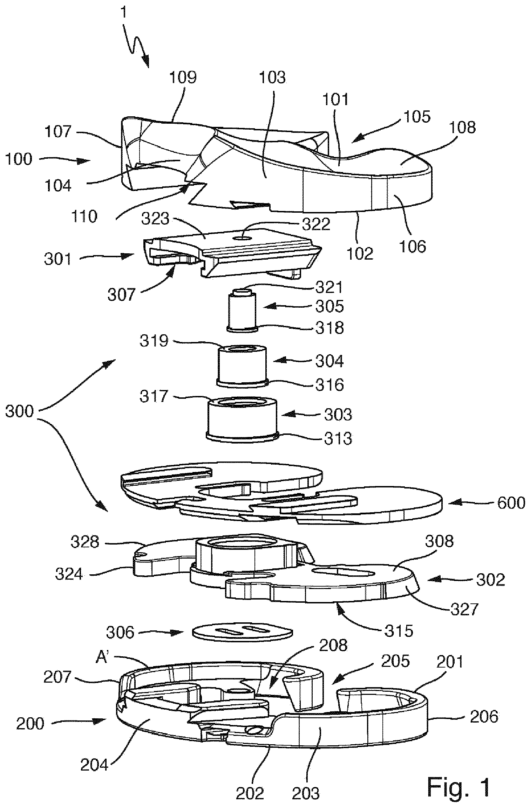

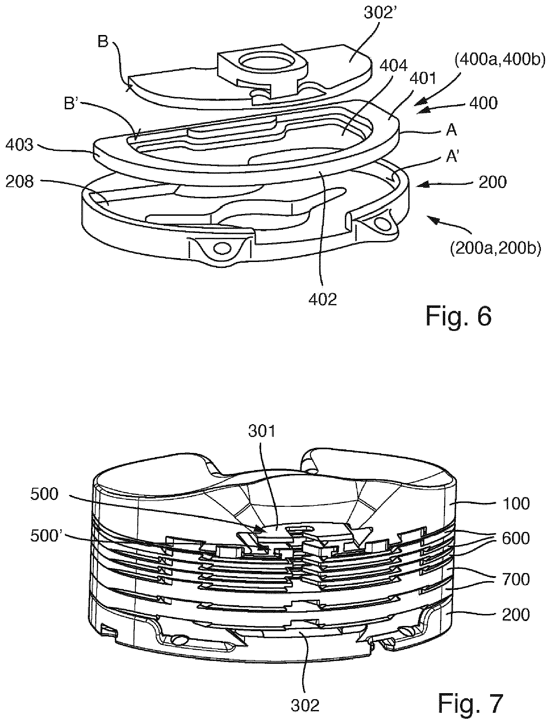

According to fig. 1, a tibial trial insert system 1 is provided for use in a knee joint replacement surgery. The tibial trial insert system 1 comprises a bearing component 100, a plate component 200, a spacing adjustment assembly 300 and a plurality of shims 500, 500', 600, 700, 720, 740 (cf. figs. 7 to 15d), wherein fig. 1 only shows one exemplary shim 600 of said plurality of shims 500, 500', 600, 700, 720, 740.

The bearing component 100 has a superior articulating surface 101, an opposing inferior surface 102 and a peripheral wall 103 extending from the inferior surface 102 to the superior articulating surface 101. The bearing component 100 further includes an anterior side 104, a posterior side 105, a lateral side 106 and a medial side 107. The superior articulating surface 101 is configured to articulate with natural or prosthetic condyles of a distal femur and includes a lateral articulating surface portion 108 and a medial articulating surface portion 109.

The plate component 200 has a superior surface 201, an opposing inferior fixation surface 202 and a peripheral wall 203 extending from the inferior fixation surface 202 to the superior surface 201. The plate component 200 further includes an anterior side 204, a posterior side 205, a lateral side 206 and a medial side 207. The inferior fixation surface 202 is configured for direct or indirect fastening to a proximal end of a tibia.

The spacing adjustment assembly 300 is configured to be arrangeable between the inferior surface 102 of the bearing component 100 and the superior surface 201 of the plate component 200. The spacing adjustment assembly 300 comprises a superior connector element 301 configured to removably engage with the bearing component 100 and an inferior base element 302 configured to removably engage with the plate component 200. The spacing adjustment assembly 300 further comprises a coupling arrangement 303, 304, 305, 306 movably coupling the connector element 301 with the base element 302 in proximal/distal direction and to a limited extent. In other words, the connector element 301 and the base element 302 are movably and captively coupled to each other by means of the coupling arrangement 303, 304, 305, 306.

The spacing adjustment assembly 300 is intended for adjusting a relative proximal/distal spacing between the bearing component 100 and the plate component 200. In other words, the spacing adjustment assembly 300 is intended to position the superior articulating surface 101 and/or the bearing component 100 in different height levels relative to the plate component 200, in particular relative to the inferior fixation surface 202. Such a spacing or height adjustment is required for trial reposition in knee joint replacement surgery. Said trial reposition is a preceding operation step of the actual knee joint replacement, wherein sizes, dimensions and/or shapes of the tibial and femoral implant components required for a functional replacement of the knee joint are determined. This application related background of the tibial trial insert system 1 is well-known to a person skilled in the art. Therefore, no further explanations are needed in that respect.

With respect to said height and/or spacing adjustment, each shim 500, 500', 600, 700, 720, 740 of said plurality of shims is configured to be slidable between an inferior surface 307 of the connector element 301 and a superior surface 308 of the base element 302.

For that purpose, a single shim or a plurality of stacked shims can be inserted between the inferior surface 307 and the superior surface 308. In other words, two or more of the plurality of shims can be used in combination to provide for an additional spacing between the bearing component 100 and the plate component 200.

For adjusting the proximal/distal spacing, the operating surgeon arranges the spacing adjustment assembly 300 between the bearing component 100 and the plate component 200 and connects the connector element 301 and the base element 302 to the bearing component 100 and the plate component 200, respectively, in a manner described in more detail below. The actual spacing adjustment is achieved by inserting at least one of the shims 500, 500', 600, 700, 720, 740 between the inferior surface 307 and the superior surface 308.

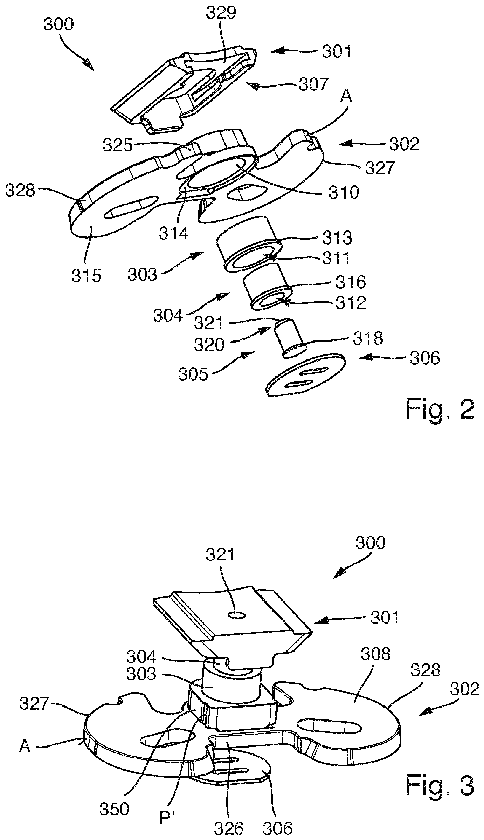

The coupling arrangement 303, 304, 305, 306 forms a telescopic mechanism (fig. 2, 3) which is extendable in proximal direction and retractable in distal direction. The telescopic mechanism is configured to movably and captively couple the connector element 301 with the base element 302.

In the embodiment shown the telescopic mechanism comprises a first cylinder element 303, a second cylinder element 304 and a third cylinder element 305. The first, second and third cylinder elements 303, 304, 305 form a three-stage telescopic cylinder. In embodiments not illustrated in the drawings, the telescopic mechanism comprises a different number of cylinder elements, forming for example a two-, four-, five- or six-stage telescopic cylinder with an according number of cylinder elements.

The first cylinder element 303 is slidably received in a bore 310 of the base element 302. The second cylinder element 304 is slidably received in a bore 311 of the first cylinder element 303. The third cylinder element 305 is slidably received in a bore 312 of the second cylinder element.

The bores 310, 311, 312 extend coaxially and in the proximal/distal direction. Moreover, the bores 310, 311, 312 are configured as through-holes.

The first cylinder element 303 has a distal radial collar 313. The bore 310 of the base element 302 has a proximal radial collar (not specified in more detail). The radial distal collar 313 of the first cylinder element 303 and the proximal radial collar of the bore 310 form an end-stop for limiting proximal displacement of the first cylinder element 303 within the bore 310. For limiting distal displacement, the coupling arrangement 303, 304, 305, 306 comprises a plug element 306. The plug element 306 forms a distal closure of the bore 310. In the illustrated embodiment the plug element 306 has a disk-like form and is fitted within a recess 314 which is countersunk in proximal direction into an inferior surface 315 of the base element 302. Moreover, the plug element 306 is connected to the base element 302 by means of a weld connection (not specified in more detail).

The second cylinder element 304 has a distal radial collar 316. The first cylinder element 303 has a proximal radial collar 317 (fig. 1). The distal radial collar 316 and the proximal radial collar 317 form an end-stop for limiting proximal displacement of the second cylinder element 304 within the bore 311. Distal displacement of the second cylinder element 304 is limited by means of the plug element 306.

The third cylinder element 305 has a distal radial collar 318. The second cylinder element 304 has a proximal radial collar 319 (fig. 1). The distal radial collar 318 and the proximal radial collar 319 form an end-stop for limiting proximal displacement of the third cylinder element 305 within the bore 312. Distal displacement of the third cylinder element 305 is limited by means of the plug element 306. A proximal end 320 of the third cylinder element 305 is fixedly joined to the inferior surface 307 of the connector element 301. In the illustrated embodiment, a welded connection is provided for that purpose between a pin portion 321 towering from the proximal end 320 in proximal direction and a pin hole 322 of the connector element 301. The pin hole 322 extends between the inferior surface 307 and a superior surface 323 of the connector element 301.

As a result, the connector element 301 is movable yet captively coupled to the base element 302 by means of the coupling arrangement 303, 304, 305, 306.

The telescopic mechanism is configured to prevent a relative rotation between the bearing component 100 and the plate component 200 about a proximal/distal axis. To this end, the first, second and third cylinder element 303, 304, 305 each have a rotationally asymmetrical cross-section. The same holds for the bores 310, 311, 312. The rotationally asymmetrical cross-sections prevent relative rotation between the first cylinder element 303 and the base element 302, between the second cylinder element 304 and the first cylinder element 303 as well as between the third cylinder element 305 and the second cylinder element 304. In the illustrated embodiment, the cylinder elements 303, 304, 305 and the bores 310, 311, 312 each comprise an oval cross-section.

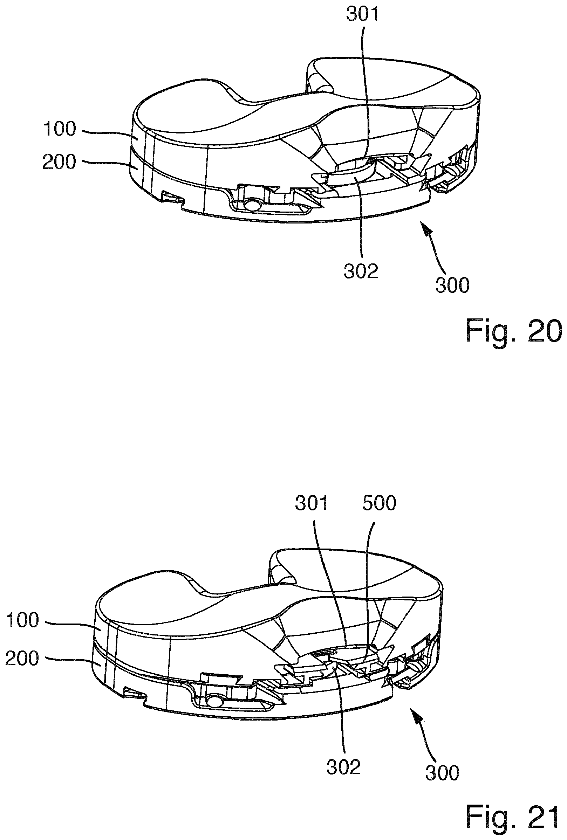

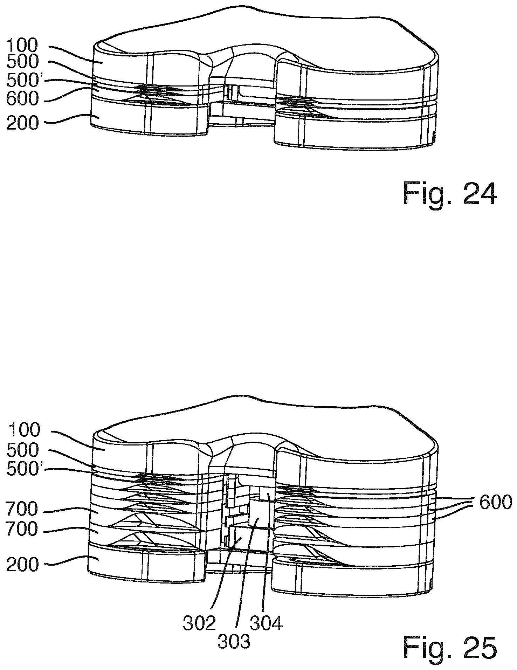

In the illustrated embodiment, the telescopic mechanism is extendable between 0 mm (fig. 20) and 16 mm (fig. 7, 25).

For removable engagement with the connector element 301, the bearing component 100 comprises an engagement portion 110. The engagement portion 110 is countersunk into the inferior surface 102 of the bearing component 100 in the proximal direction. In the embodiment as illustrated, the engagement portion 110 is designed in the form of a dovetail guide. The latter is plug-in connectable to the connector element 301 in anterior/posterior direction. In the plugged-together state, relative movement between the connector element 301 and the bearing component 100 is inhibited at least in proximal/distal direction and in medial/lateral direction.

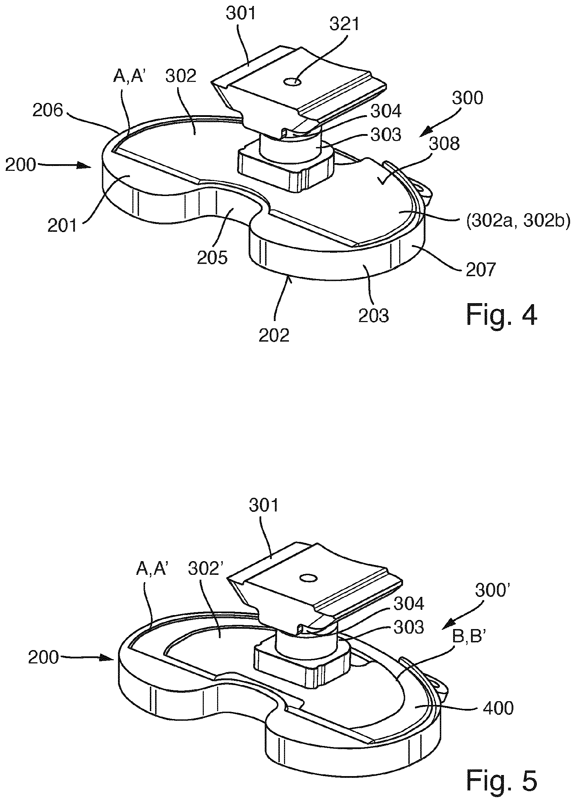

The base element has a peripheral wall 324 extending between the inferior surface 315 and the superior surface 308. Moreover, the base element has an anterior side 325, a posterior side 326, a lateral side 327, and a medial side 328. The peripheral wall 324 has an outer contour A. For removable engagement with the base element 302, the plate component 200 has a receiving recess 208 which is countersunk in the distal direction into the superior surface 201. The receiving recess 208 has an inner contour A' which is complementary to the outer contour A of the base element 302. In a condition received in the receiving recess 208, the base element 302 is restrained in anterior/posterior direction and lateral/medial direction. Such a condition is illustrated in fig. 4.

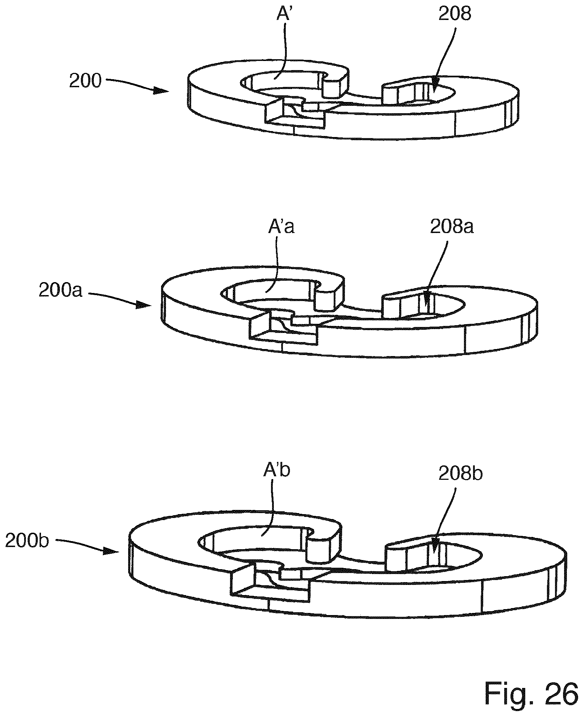

In the embodiment as illustrated, the tibial trial insert system 1 comprises a set of different sized plate components 200, 200a, 200b (fig. 26). The different sized plate components 200, 200a, 200b differ with respect to the sizes of their receiving recesses 208, 208a, 208b and, thus, with respect to the shapes and sizes of the inner contours A', A'a, A'b. In embodiments not illustrated in the drawings, the set comprises different numbers of different sized plate components, for example merely two or more than the depicted three, for example four or five, different sized plate components.

In order to accommodate for the different sized plate components 200, 200a, 200b, the tibial trial insert system 1 can comprise an according set of different spacing adjustment assemblies having different sized base elements. Each of the base elements is associated with one of the plate components 200, 200a, 200b and configured to removably engage with said plate component. In other words, the set of different spacing adjustment assemblies has base elements 302, 302a, 302b differing with respect to their size and/or shape. Said different base elements 302, 302a, 302b are symbolized in fig. 4 by one and the same base element, for the sake of brevity.

Alternatively, the tibial trial insert system 1 comprises at least one adapter element 400 that allows using a single spacing adjustment assembly 300' having a base element 302' for different sized plate components (fig. 5, 6). The adapter element 400 has a superior surface 401, an opposing inferior surface 402 and a peripheral wall 403 extending between the inferior surface 402 and the superior surface 401. In the embodiment as illustrated, the peripheral wall 403 has an outer contour A that is complementary to the inner receiving contour A' of the plate component 200. Moreover, the adapter element 400 has a receiving recess 404 which is countersunk into the superior surface 401 in distal direction. The receiving recess 404 has an inner contour B' that is complementary to an outer contour B of the base element 302'. In order to accommodate for the set of different sized plate components 200, 200a, 200b, the tibial trial insert system 1 can comprise a set of different sized adapter elements 400, 400a, 400b (fig. 6), each of the adapter elements 400, 400a, 400b being associated with one of the plate components 200, 200a, 200b. For the sake of brevity the different sized adapter elements 400, 400a, 400b are symbolized by means of one and the same adapter element within fig. 4. The different sized adapter elements 400, 400a, 400b differ with respect to the size and/or shape of their outer contours. Apart from that, the different adapter elements 400, 400a, 400b are identical.

For adjusting the height of the spacing adjustment assembly 300, the tibial trial insert system 1 comprises said plurality of shims, six shims 500, 500', 600, 700, 720, 740 of the plurality of shims being illustrated as an example with reference to fig. 7 to fig. 15d. The shims depicted in fig. 7 to fig. 15d can also be referred to as first shim 500, second shim 600, third shim 700, fourth shim 720 and fifth shim 740. The plurality of shims comprises at least one first shim 500, one second shim 600, one third shim 700, one fourth shim 720 and one fifth shim 740. Preferably, the plurality of shims comprises several first, second, third and fourth shims. Said shims differ with respect to their thicknesses and comprise different matching portions and different complementary matching portions:
The first shim 500 has a first thickness t1. The second shim 600 has a second thickness t2. The third shim 700 has a third thickness t3. The fourth shim 720 has a fourth thickness t4. The fifth shim 740 has a fifth thickness t5.

In the embodiment as illustrated, the second thickness t2 is twice the first thickness t1. The third thickness t3 is twice the second thickness t2. In the present case, 1 mm is provided as the first thickness t1. The second thickness t2 and the third thickness t3 are accordingly 2 mm and 4 mm, respectively. Moreover, the fourth thickness t4 is 6 mm and the fifth thickness t5 is 8 mm.

The embodiment as illustrated comprises is a further shim 500' having the same first thickness t1 as the first shim 500. Since the first shim 500 and the further shim 500' have the same thickness, they can also be referred to as first shim of first type 500 and first shim of second type 500'.

In order to prevent a stacking of the shims in a non-defined stacking order, the shims comprise said different matching portions and/or said different complementary matching portions:
The first shim 500 comprises a first matching portion 501. The first matching portion 501 is disposed at an anterior side of the first shim 500. The first matching portion 501 is configured for matched fitting with a complementary matching portion 329 of the connector element 301 (fig. 2). The complementary matching portion 329 is disposed at the inferior surface 307 of the connector element 301 and forms a slot for receiving the first matching portion 501.

The further shim 500' comprises a further matching portion 501'. The further matching portion 501' is disposed at an anterior side of the second shim 500' and configured for matched fitting with a first complementary matching portion 502 of the first shim 500. The first complementary matching portion 502 forms a slot for receiving the further matching portion 501'.

The size and shape of the first matching portion and the further matching portion ensures that the first shim 500 can only be used in a first position, i.e. directly underneath the connector element 301. Correspondingly the further shim 500' can only be inserted into a second position, i.e. directly underneath the first shim 500.

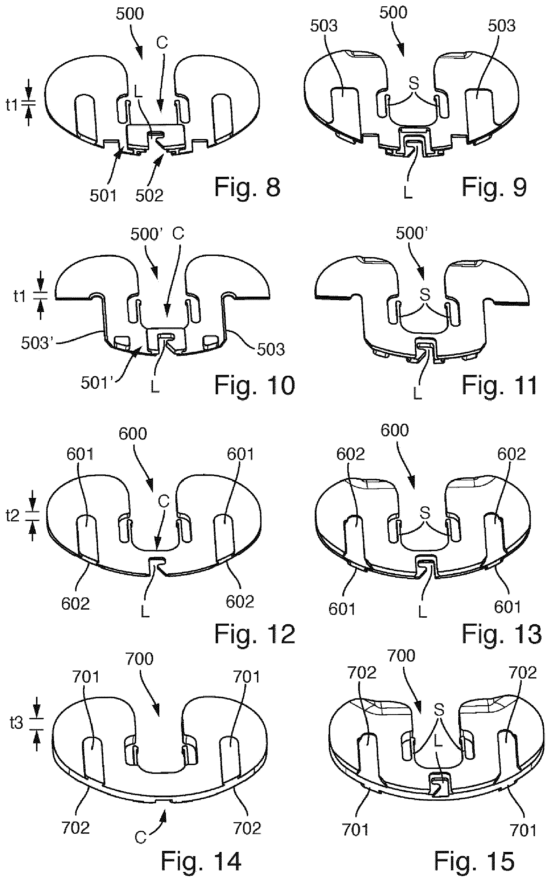

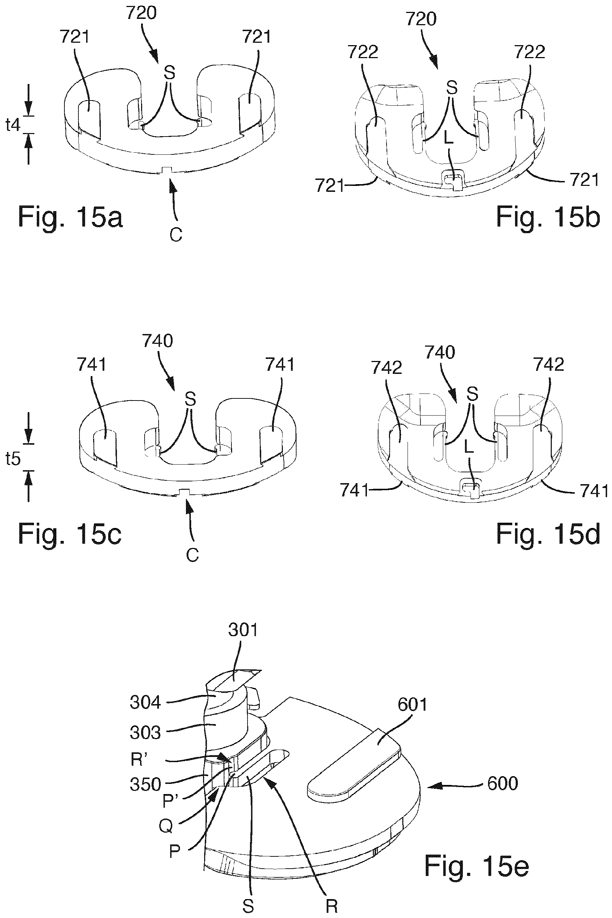

The second shim 600 has a second matching portion 601 and the third shim 700 has a third matching portion 701. In the embodiment as illustrated, the second and third shim 600, 700 both have two matching portions. Each of the matching portions 601, 701 forms a protrusion protruding from a superior surface of the according shim. The second shim 600 can be used in a second position underneath the first shim 500. Alternatively, the second shim 600 can be used in a third position underneath the further shim (i.e., the first shim of second type) 500', the latter being positioned underneath the first shim 500. Alternatively, the second shim 600 can be used in a first position directly underneath the connector element 301. In the second position the second matching portions 601 are fitted matching with recesses 503, which are countersunk into an inferior surface of the first shim 500 in proximal direction (fig. 9). In the third position the second matching portions 601 are fitted matching with medial and lateral sides 503' of the further shim 500'. Moreover, the second matching portions 601 can be matched with second complementary matching portions 602 of another second shim 600 (fig. 13) or with third complementary matching portions 702 of the third shim (fig. 15). The second and third complementary matching portions 602, 702 each are in the form of a recess, which is countersunk into the inferior surface of the respective shim in proximal direction.

The third shim 700 can be positioned just like the second shim 600.

The same holds analogously with respect to design and/or function of the fourth shim 720 and the fifth shim 740: The fourth shim 720 comprises fourth matching portions 721 and fourth complementary matching portions 722. The fifth shim 740 comprises fifth matching portion 741 and complementary fifth matching portions 742. Using one of the fourth and/or fifth shims 720, 740 helps the operating surgeon to avoid lengthy stacking procedures for achieving a desired thickness. The different design of the afore-mentioned matching portions and complementary matching portions prevents stacking orders of the shims that do not correspond to the proximal/distal heights, i.e., thicknesses, available on the permanent prosthesis. In other words, well-defined stacking orders have to be respected to set up the different thicknesses. It is the different shape and/or size of the matching and complementary matching portions that helps the operating surgeon to stay within said stacking orders.

Figs. 20 to 25 depict different configurations of the tibial trial insert system 1 with different adjustments of the relative proximal distal spacing between the bearing component 100 and the plate component 200. In other words, the tibial trial insert system 1 is shown in different configurations having different overall heights.

A first configuration (fig. 20) has a first height. To achieve the first height, none of the shims 500, 500', 600, 700, 720, 740 has to be used. Solely the spacing adjustment assembly 300 is disposed between the inferior surface of the bearing component 100 and the superior surface of the plate component 200.

A second configuration (fig. 21) has a second height. The second height is achieved using a single first shim 500. The first shim 500 is positioned directly underneath the connector element 301, i.e., in the first position.

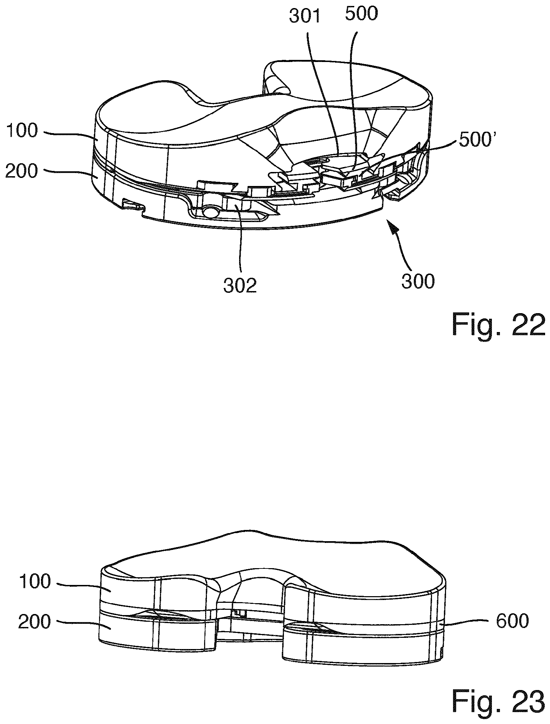

A third configuration (fig. 22) has a third height. The third height is achieved using one first shim 500 and one further shim 500'. The first shim 500 is in the first position. The further shim 500' is in the second position directly underneath the first shim 500.

Alternatively, the third height as depicted in fig. 22 can also be achieved by using a single second shim 600 in the first position (fig. 23). The configuration depicted in fig. 23 can also be denoted as fourth configuration.

A fifth configuration (fig. 24) has a fifth height. The fifth height is achieved using one first shim 500 in the first position, one further shim 500' in the second position and one second shim 600 in the third position.

A sixth configuration (fig. 7, 25) has a sixth height. The sixth height is achieved using one first shim 500 in the first position, one further shim 500' in the second position, three second shims 600 in the third, fourth and fifth position and two third shims 700 in the sixth and seventh position.

In the first configuration the telescopic mechanism is completely retracted in distal direction (fig. 20). In the configuration depicted in fig. 7 and fig. 25 the telescopic mechanism is completely extended in proximal direction, preventing the operating surgeon from sliding further shims between the plate component 200 and the bearing component 100, thereby setting a height that is not available on the final implants and/or prostheses.

In order to prevent an unintended removal from, e.g., one of the above-mentioned configurations each of the shims 500, 500', 600, 700, 720, 740 comprises a spring-loaded latching portion R. Each of the latching portions R is configured for releasable form- and/or force-fitting connection with a complementary latching portion R' of the spacing adjustment assembly 300. The latching portions R and the complementary latching portion R' form a releasable latching connection Q (fig. 15e). Said latching connection Q helps to avoid an unintended removal of the according shim, e.g., the third shim 600 as depicted in fig. 15e. Moreover, the latching connection Q mechanically stabilizes the shims in their stacked configuration and thereby the whole tibial trial insert system 1.

In the embodiment as illustrated each of the latching portions R comprises two spring elements S in the form of an elongated beam spring. The spring elements S each comprise a latching protrusions P for latching interaction with the complementary latching portion R'. The spring elements S are elastically deformable to generate said spring load onto the respective latching protrusion P. The protrusions P are disposed at an end portion of the respective spring element S.

In the embodiment as illustrated the complementary latching portion R' is a portion of the base element 302 and disposed on and/or in an outer wall of a boss section 350, wherein the boss section 350 of the base element 302 also comprises the cylindrical bore 310. The complementary latching portion R' comprises two latching recesses P'. Each of the latching recesses P' is configured for receiving one of the latching protrusions P.

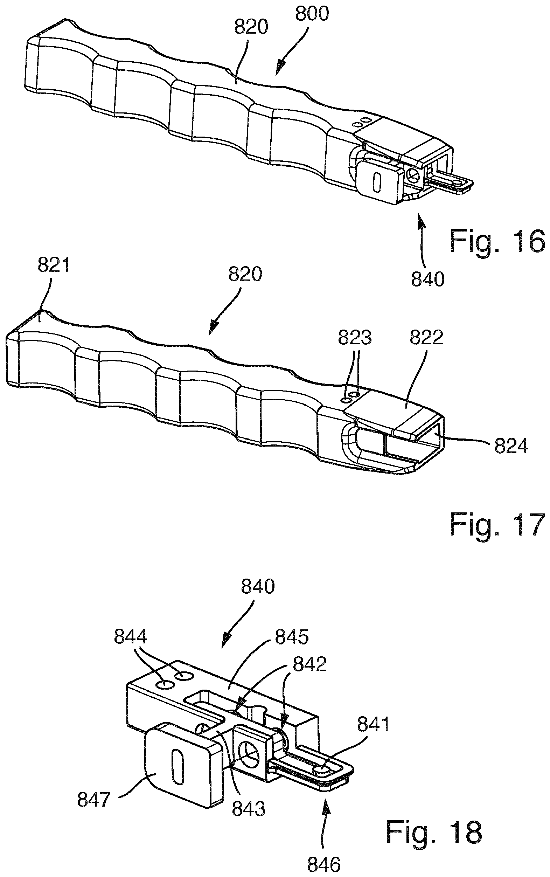

In the embodiment as illustrated, the trial insert system 1 further comprises a shim handling instrument 800 having an instrument shaft 820 and a coupling mechanism 840 (figs. 16, 17, 18). The instrument shaft 820 is extended between a proximal end 821 and a distal end 822. The coupling mechanism 840 is disposed at the distal end 822.

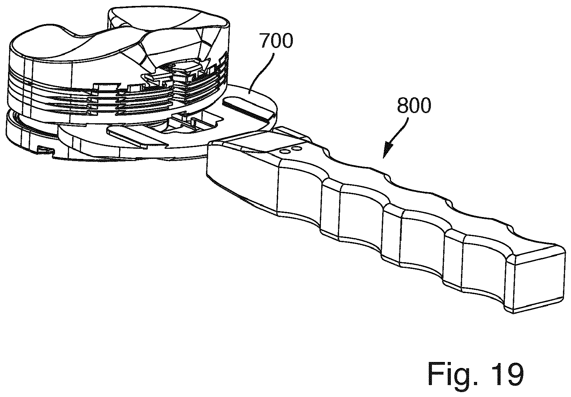

The coupling mechanism 840 is configured to releasably couple to an anterior coupling portion C of each of the plurality of shims 500, 500', 600, 700, 720, 740 (figs. 8 to 15d). The coupling mechanism 840 comprises a coupling finger 841. The coupling finger 841 extends in proximal/distal direction (fig. 18, 19) and is spring-loaded by means of at least one spring element 842 in medial/lateral direction. In the embodiment as illustrated, the coupling mechanism 840 comprises two spring elements 842 that act on the coupling finger 841. The coupling portions C each comprise an L-shaped coupling slot L for positively receiving the coupling finger 841. The L-shaped coupling slots L each extend in anterior/posterior and medial/lateral direction. The anterior coupling portions C and the L-shaped coupling slots L have an identical shape and/or size for each shim.

The coupling mechanism 840 further comprises a housing 845 in which the coupling finger 841 is mounted movable in medial/lateral direction. Also the spring elements 842 are mounted within the housing 845. The housing 845 is mounted within a cavity 824 disposed distally at the instrument shaft 820. The instrument shaft 820 and the housing 845 have pin holes 823, 824 for fixing the housing 845 within the mounting cavity 824.

In the embodiment as illustrated, the housing 845 has a distal coupling protrusion 846. The coupling portions C of the shims are each configured to form-fittingly receive the coupling protrusion 846.

Fig. 19 illustrates an exemplary handling of a third shim 700 by means of the shim handling instrument 800. For coupling the third shim 700 with the shim handling instrument 800, the coupling protrusion 846 is inserted into the coupling portion C in anterior/posterior direction with respect to the situation depicted in fig. 19. Before inserting the coupling protrusion 846 into the coupling portion C, the operating surgeon manually moves the coupling finger 841 in lateral direction by means of pressing a button 847. In the embodiment as illustrated, the button 847 is integral with the coupling finger 841 and, thus, spring-loaded in lateral direction. Having pressed the button 847, the coupling finger 841 can enter the L-shaped coupling slot L. When depressing the button 847, the spring elements 842 push the coupling finger 841 in lateral direction, whereby the coupling finger 841 enters the medial/lateral portion of the L-shaped coupling slot L. In this position the third shim 700 is securely coupled with the shim handling instrument 800.

A decoupling of the third shim 700 is achieved in kinematically reverse order.

Moreover, it is important to observe that insertion of a shim of the plurality of shims 500, 500', 600, 700, 720, 740 is possible in the last position only, i.e. directly above the base element 302 as depicted in fig. 19. This is due to the specific design of the shims 500, 500', 600, 700, 720, 740 and/or their matching portions and complementary matching portions. Put in other words it is not possible to insert a shim between two other shims.

## Claims

1. Tibial trial insert system (1), comprising:
a bearing component (100) having a superior articulating surface (101) and an inferior surface (102);
a plate component (200) having a superior surface (201) and an inferior fixation surface (202);
a spacing adjustment assembly (300) configured to be arrangeable between the inferior surface (102) of the bearing component (100) and the superior surface (201) of the plate component (200), the spacing adjustment assembly (300) having at least one superior connector element (301) configured to removably engage with the bearing component (100), at least one inferior base element (302) configured to removably engage with the plate component (200), and having a coupling arrangement (303, 304, 305, 306) movably coupling the connector element (301) with the base element (302) in proximal/distal direction and to a limited extent;
a plurality of shims (500, 500', 600, 700, 720, 740), each shim (500, 500', 600, 700, 720, 740) configured to be slidable between an inferior surface (307) of the connector element (301) and a superior surface (308) of the base element (302) to adjust a proximal/distal height of the spacing adjustment assembly (300), in order to thereby adjust a relative proximal/distal spacing between the bearing component (100) and the plate component (200).

2. Tibial trial insert system (1) according to claim 1, **characterized in that** the coupling arrangement (303, 304, 305, 306) forms a telescopic mechanism which is extendable in proximal direction and retractable in distal direction.

3. Tibial trial insert system (1) according to claim 2, **characterized in that** the telescopic mechanism comprises at least a first cylinder element (303) and a second cylinder element (304), wherein the first cylinder element (303) is slidably received in a bore (310) of the base element (302), wherein the second cylinder element (304) is slidably received in a bore (311) of the first cylinder element (303), and wherein the second cylinder element (304) is at least indirectly connected to the inferior surface (307) of the connector element (301).

4. Tibial trial insert system (1) according to claim 3, **characterized in that** the first cylinder element (303) and the second cylinder element (304) each have a rotationally asymmetrical, preferably oval, cross-section, in order to thereby prevent a rotation about a proximal/distal axis.

5. Tibial trial insert system (1) according to any of claims 2 to 4, **characterized in that** the telescopic mechanism is extendable between 0 mm and 16 mm, inclusive.

6. Tibial trial insert system (1) according to any of the preceding claims, further comprising a set of different sized plate components (200, 200a, 200b) and a set of different sized adapter elements (400, 400a, 400b), each of the adapter elements (400, 400a, 400b) being associated with one of the plate components (200, 200a, 200b) and configured to removably engage with one of the plate components (200, 200a, 200b) and with the base element (302) of the spacing adjustment assembly (300).

7. Tibial trial insert system (1) according to any of claims 1 to 5, further comprising a set of different sized plate components (200, 200a, 200b) and a set of different spacing adjustment assemblies having different sized base elements (302, 302a, 302b), each of the base elements (302, 302a, 302b) being associated with one of the plate components (200, 200a, 200b) and configured to removably engage with the one of the plate components (200, 200a, 200b).

8. Tibial trial insert system (1) according to any of the preceding claims, **characterized in that** the plurality of shims (500, 500', 600, 700, 720, 740) can be stacked one above another and comprises different shims having different thicknesses (t1, t2, t3, t4, t5) and different matching portions (501, 501', 601, 701, 721, 741), each of the matching portions (501, 501', 601, 701, 721, 741) being configured for matching with a different complementary matching portion of the plurality of shims (500, 500', 600, 700, 720, 740) and/or the spacing adjustment assembly (300), in order to thereby prevent a stacking of the plurality of shims (500, 500', 600, 700, 720, 740) in a non-defined stacking order.

9. Tibial trial insert system (1) according to claim 8, **characterized in that** the different shims of the plurality of shims (500, 500', 600, 700, 720, 740) each comprise a spring-loaded latching portion (R), each of the latching portions (R) being configured for releasable form- and/or force-fitting connection with a complementary latching portion (R') of the spacing adjustment assembly (300).

10. Tibial trial insert system (1) according to claim 8 or 9, **characterized in that** the different thicknesses (t1, t2, t3, t4, t5) range between 1 mm and 8 mm, inclusive.

11. Tibial trial insert system (1) according to any of the preceding claims, **characterized in that** the plurality of shims (500, 500', 600, 700, 720, 740) comprises at least one first shim (500, 500') having a first thickness (t1), preferably of 1 mm, at least one second shim (600) having a second thickness (t2), preferably of 2 mm, at least one third shim (700) having a third thickness (t3), preferably of 4 mm, at least one fourth shim (720) having a fourth thickness (t4), preferably 6 mm, and at least one fifth shim (740) having a fifth thickness (t5), preferably 8 mm.

12. Tibial trial insert system (1) according to any of the preceding claims, further comprising a shim handling instrument (800) having an instrument shaft (820) and a coupling mechanism (840) distally disposed on the instrument shaft (820), the coupling mechanism (840) being configured to releasably couple to an anterior coupling portion (C) of each of the plurality of shims (500, 500', 600, 700, 720, 740).

13. Tibial trial insert system (1) according to claim 12, wherein the coupling mechanism (840) comprises a coupling finger (841) extending in proximal/distal direction and being spring-loaded in medial/lateral direction, and wherein the coupling portions (C) each comprise an L-shaped coupling slot (L) for positively receiving the coupling finger (841), the L-shaped coupling slot (L) extending in anterior/posterior and medial/lateral direction.

## Patentansprüche

1. Tibiales Probeeinsatzsystem (1), umfassend:
eine Lagerkomponente (100) mit einer oberen Artikulationsfläche (101) und einer unteren Fläche (102), eine Plattenkomponente (200) mit einer oberen Fläche (201) und einer unteren Fixierungsfläche (202),
eine Abstandsverstellbaugruppe (300), die dazu ausgestaltet ist, dass sie zwischen der unteren Fläche (102) der Lagerkomponente (100) und der oberen Fläche (201) der Plattenkomponente (200) angeordnet werden kann, wobei die Abstandsverstellbaugruppe (300) mindestens ein oberes Verbinderelement (301), das dazu ausgestaltet ist, entfernbar mit der Lagerkomponente (100) in Eingriff zu kommen, mindestens ein unteres Basiselement (302), das dazu ausgestaltet ist, entfernbar mit der Plattenkomponente (200) in Eingriff zu kommen, und eine Koppelanordnung (303, 304, 305, 306) hat, die das Verbinderelement (301) in proximal/distaler Richtung und in begrenztem Maße beweglich mit dem Basiselement (302) koppelt,
eine Mehrzahl von Abstandsstücken (500, 500', 600, 700, 720, 740), wobei jedes Abstandsstück (500, 500', 600, 700, 720, 740) zwischen einer unteren Fläche (307) des Verbinderelements (301) und einer oberen Fläche (308) des Basiselements (302) verschiebbar ausgestaltet ist, um eine proximal/distale Höhe der Abstandsverstellbaugruppe (300) zu verstellen, um dadurch einen proximal/distalen Relativabstand zwischen der Lagerkomponente (100) und der Plattenkomponente (200) zu verstellen.

2. Tibiales Probeeinsatzsystem (1) nach Anspruch 1, **dadurch gekennzeichnet, dass** die Koppelanordnung (303, 304, 305, 306) einen teleskopischen Mechanismus bildet, der in proximaler Richtung ausfahrbar und in distaler Richtung einfahrbar ist.

3. Tibiales Probeeinsatzsystem (1) nach Anspruch 2, **dadurch gekennzeichnet, dass** der teleskopische Mechanismus mindestens ein erstes Zylinderelement (303) und ein zweites Zylinderelement (304) umfasst, wobei das erste Zylinderelement (303) verschiebbar in einer Bohrung (310) des Basiselements (302) aufgenommen ist, wobei das zweite Zylinderelement (304) verschiebbar in einer Bohrung (311) des ersten Zylinderelements (303) aufgenommen ist und wobei das zweite Zylinderelement (304) mindestens indirekt mit der unteren Fläche (307) des Verbinderelements (301) verbunden ist.

4. Tibiales Probeeinsatzsystem (1) nach Anspruch 3, **dadurch gekennzeichnet, dass** das erste Zylinderelement (303) und das zweite Zylinderelement (304) jeweils einen rotationsmäßig asymmetrischen, vorzugsweise ovalen, Querschnitt haben, um dadurch eine Drehung um eine proximal/distale Achse zu verhindern.

5. Tibiales Probeeinsatzsystem (1) nach einem der Ansprüche 2 bis 4, **dadurch gekennzeichnet, dass** der teleskopische Mechanismus zwischen einschließlich 0 mm und 16 mm ausfahrbar ist.

6. Tibiales Probeeinsatzsystem (1) nach einem der vorhergehenden Ansprüche, ferner umfassend einen Satz unterschiedlich bemessener Plattenkomponenten (200, 200a, 200b) und einen Satz unterschiedlich bemessener Adapterelemente (400, 400a, 400b), wobei jedes der Adapterelemente (400, 400a, 400b) einer der Plattenkomponenten (200, 200a, 200b) zugeordnet und dazu ausgestaltet ist, mit einer der Plattenkomponenten (200, 200a, 200b) und mit dem Basiselement (302) der Abstandsverstellbaugruppe (300) entfernbar in Eingriff zu kommen.

7. Tibiales Probeeinsatzsystem (1) nach einem der Ansprüche 1 bis 5, ferner umfassend einen Satz unterschiedlich bemessener Plattenkomponenten (200, 200a, 200b) und einen Satz unterschiedlicher Abstandsverstellbaugruppen mit unterschiedlich bemessenen Basiselementen (302, 302a, 302b), wobei jedes der Basiselemente (302, 302a, 302b) einer der Plattenkomponenten (200, 200a, 200b) zugeordnet und dazu ausgestaltet ist, mit einer der Plattenkomponenten (200, 200a, 200b) entfernbar in Eingriff zu kommen.

8. Tibiales Probeeinsatzsystem (1) nach einem der vorhergehenden Ansprüche, **dadurch gekennzeichnet, dass** die Mehrzahl von Abstandsstücken (500, 500', 600, 700, 720, 740) übereinandergestapelt werden können und verschiedene Abstandsstücke mit verschiedenen Dicken (t1, t2, t3, t4, t5) und verschiedenen übereinstimmenden Abschnitten (501, 501', 601, 701, 721, 741) umfassen, wobei jeder der übereinstimmenden Abschnitte (501, 501', 601, 701, 721, 741) zum Übereinstimmen mit einem anderen komplementären übereinstimmenden Abschnitt der Mehrzahl von Abstandsstücken (500, 500', 600, 700, 720, 740) und/oder der Abstandsverstellbaugruppe (300) ausgestaltet ist, um dadurch ein Stapeln der Mehrzahl von Abstandsstücken (500, 500', 600, 700, 720, 740) in einer nicht definierten Stapelordnung zu verhindern.

9. Tibiales Probeeinsatzsystem (1) nach Anspruch 8, **dadurch gekennzeichnet, dass** die verschiedenen Abstandsstücke der Mehrzahl von Abstandsstücken (500, 500', 600, 700, 720, 740) jeweils einen gefederten Rastabschnitt (R) umfassen, wobei jeder der Rastabschnitte (R) für eine freigebbare form- und/oder kraftschlüssige Verbindung mit einem komplementären Rastabschnitt (R') der Abstandsverstellbaugruppe (300) ausgestaltet ist.

10. Tibiales Probeeinsatzsystem (1) nach Anspruch 8 oder 9, **dadurch gekennzeichnet, dass** die verschiedenen Dicken (t1, t2, t3, t4, t5) zwischen einschließlich 1 mm und 8 mm liegen.

11. Tibiales Probeeinsatzsystem (1) nach einem der vorhergehenden Ansprüche, **dadurch gekennzeichnet, dass** die Mehrzahl von Abstandsstücken (500, 500', 600, 700, 720, 740) mindestens ein erstes Abstandsstück (500, 500') mit einer ersten Dicke (t1) von vorzugsweise 1 mm, mindestens ein zweites Abstandsstück (600) mit einer zweiten Dicke (t2) von vorzugsweise 2 mm, mindestens ein drittes Abstandsstück (700) mit einer dritten Dicke (t3) von vorzugsweise 4 mm, mindestens ein viertes Abstandsstück (720) mit einer vierten Dicke (t4) von vorzugsweise 6 mm, und mindestens ein fünftes Abstandsstück (740) mit einer fünften Dicke (t5) von vorzugsweise 8 mm umfassen.

12. Tibiales Probeeinsatzsystem (1) nach einem der vorhergehenden Ansprüche, ferner umfassend ein Abstandsstückhandhabungsinstrument (800) mit einem Instrumentenschaft (820) und einem distal an dem Instrumentenschaft (820) angeordneten Koppelmechanismus (840), wobei der Koppelmechanismus (840) dazu ausgestaltet ist, freigebbar an einen anterioren Koppelabschnitt (C) jedes der Mehrzahl von Abstandsstücken (500, 500', 600, 700, 720, 740) zu koppeln.

13. Tibiales Probeeinsatzsystem (1) nach Anspruch 12, wobei der Koppelmechanismus (840) einen Koppelfinger (841) umfasst, der sich in proximal/distaler Richtung erstreckt und in medial/lateraler Richtung gefedert ist, und wobei die Koppelabschnitte (C) jeweils einen L-förmigen Koppelschlitz (L) zur positiven Aufnahme des Koppelfingers (841) umfassen, wobei sich der L-förmige Koppelschlitz (L) in anterior/posteriorer und medial/lateraler Richtung erstreckt.

## Revendications

1. Système d'insertion d'essai tibial (1), comprenant :
un composant de palier (100) ayant une surface d'articulation supérieure (101) et une surface inférieure (102) ;
un composant de plaque (200) ayant une surface supérieure (201) et une surface de fixation inférieure (202) ;
un ensemble de réglage d'espacement (300) configuré pour être agencé entre la surface inférieure (102) du composant de palier (100) et la surface supérieure (201) du composant de plaque (200), l'ensemble de réglage d'espacement (300) ayant au moins un élément de raccordement supérieur (301) configuré pour venir en prise de manière amovible avec le composant de palier (100), au moins un élément de base inférieur (302) configuré pour venir en prise de manière amovible avec le composant de plaque (200), et ayant un dispositif d'accouplement (303, 304, 305, 306) couplant de manière mobile l'élément de raccordement (301) avec l'élément de base (302) dans la direction proximale/distale et dans une mesure limitée ;
une pluralité de cales (500, 500', 600, 700, 720, 740), chaque cale (500, 500', 600, 700, 720, 740) étant configurée de façon à pouvoir coulisser entre une surface inférieure (307) de l'élément de raccordement (301) et une surface supérieure (308) de l'élément de base (302) afin de régler une hauteur proximale/distale de l'ensemble de réglage d'espacement (300), afin de régler ainsi un espacement proximal/distal relatif entre le composant de palier (100) et le composant de plaque (200) .

2. Système d'insertion d'essai tibial (1) selon la revendication 1, **caractérisé en ce que** le dispositif d'accouplement (303, 304, 305, 306) forme un mécanisme télescopique qui est extensible dans la direction proximale et rétractable dans la direction distale.

3. Système d'insertion d'essai tibial (1) selon la revendication 2, **caractérisé en ce que** le mécanisme télescopique comprend au moins un premier élément de cylindre (303) et un second élément de cylindre (304), le premier élément de cylindre (303) étant reçu de manière coulissante dans un alésage (310) de l'élément de base (302), le second élément de cylindre (304) étant reçu de manière coulissante dans un alésage (311) du premier élément de cylindre (303), et le second élément de cylindre (304) étant au moins indirectement raccordé à la surface inférieure (307) de l'élément de raccordement (301).

4. Système d'insertion d'essai tibial (1) selon la revendication 3, **caractérisé en ce que** le premier élément de cylindre (303) et le second élément de cylindre (304) ont chacun une section transversale asymétrique en rotation, de préférence ovale, afin d'empêcher une rotation autour d'un axe proximal/distal.

5. Système d'insertion d'essai tibial (1) selon l'une quelconque des revendications 2 à 4, **caractérisé en ce que** le mécanisme télescopique est extensible entre 0 mm et 16 mm, inclusivement.

6. Système d'insertion d'essai tibial (1) selon l'une quelconque des revendications précédentes, comprenant en outre un ensemble de composants de plaque de tailles différentes (200, 200a, 200b) et un ensemble d'éléments d'adaptateur de tailles différentes (400, 400a, 400b), chacun des éléments d'adaptateur (400, 400a, 400b) étant associé à l'un des composants de plaque (200, 200a, 200b) et configuré pour venir en prise de manière amovible avec l'un des composants de plaque (200, 200a, 200b) et avec l'élément de base (302) de l'ensemble de réglage d'espacement (300).

7. Système d'insertion d'essai tibial (1) selon l'une quelconque des revendications 1 à 5, comprenant en outre un ensemble de composants de plaque de tailles différentes (200, 200a, 200b) et un ensemble de différents ensembles de réglage de l'espacement ayant des éléments de base de tailles différentes (302, 302a, 302b), chacun des éléments de base (302, 302a, 302b) étant associé à l'un des composants de plaque (200, 200a, 200b) et configuré pour venir en prise de manière amovible avec l'un des composants de plaque (200, 200a, 200b) .

8. Système d'insertion d'essai tibial (1) selon l'une quelconque des revendications précédentes, **caractérisé en ce que** la pluralité de cales (500, 500', 600, 700, 720, 740) peuvent être empilées les unes sur les autres et comprend différentes cales ayant différentes épaisseurs (t1, t2, t3, t4, t5) et différentes parties d'adaptation (501, 501', 601, 701, 721, 741), chacune des parties d'adaptation (501, 501', 601, 701, 721, 741) étant configurées pour s'adapter à une partie d'adaptation complémentaire différente de la pluralité de cales (500, 500', 600, 700, 720, 740) et/ou de l'ensemble de réglage d'espacement (300), afin d'empêcher l'empilement de la pluralité de cales (500, 500', 600, 700, 720, 740) dans un ordre d'empilement non défini.

9. Système d'insertion d'essai tibial (1) selon la revendication 8, **caractérisé en ce que** les différentes cales de la pluralité de cales (500, 500', 600, 700, 720, 740) comprennent chacune une partie d'encliquetage à ressort (R), chacune des parties d'encliquetage (R) étant configurée pour un raccordement de forme et/ou par force libérable avec une partie d'encliquetage complémentaire (R') de l'ensemble de réglage d'espacement (300).

10. Système d'insertion d'essai tibial (1) selon la revendication 8 ou 9, **caractérisé en ce que** les différentes épaisseurs (t1, t2, t3, t4, t5) sont comprises entre 1 mm et 8 mm, inclusivement.

11. Système d'insertion d'essai tibial (1) selon l'une quelconque des revendications précédentes, **caractérisé en ce que** la pluralité de cales (500, 500', 600, 700, 720, 740) comprend au moins une première cale (500, 500') ayant une première épaisseur (t1), de préférence de 1 mm, au moins une deuxième cale (600) ayant une deuxième épaisseur (t2), de préférence de 2 mm, au moins une troisième cale (700) ayant une troisième épaisseur (t3), de préférence de 4 mm, au moins une quatrième cale (720) ayant une quatrième épaisseur (t4), de préférence de 6 mm, et au moins une cinquième cale (740) ayant une cinquième épaisseur (t5), de préférence de 8 mm.

12. Système d'insertion d'essai tibial (1) selon l'une quelconque des revendications précédentes, comprenant en outre un instrument de manipulation de cale (800) ayant un arbre d'instrument (820) et un mécanisme d'accouplement (840) agencé distalement sur l'arbre d'instrument (820), le mécanisme d'accouplement (840) étant configuré pour se coupler de manière amovible à une partie d'accouplement antérieure (C) de chacune de la pluralité de cales (500, 500', 600, 700, 720, 740).

13. Système d'insertion d'essai tibial (1) selon la revendication 12, le mécanisme d'accouplement (840) comprenant un doigt d'accouplement (841) s'étendant dans la direction proximale/distale et étant chargé par ressort dans la direction médiale/latérale, et les parties d'accouplement (C) comprenant chacune une fente de parties d'accouplement en forme de L (L) pour recevoir positivement le doigt d'accouplement (841), la fente d'accouplement en forme de L (L) s'étendant dans la direction antérieure/postérieure et médiale/latérale.
